# EUROPÄISCHE PATENTSCHRIFT

(11) **EP 3 536 181 B1**
(45) Veröffentlichungstag und Bekanntmachung des Hinweises auf die Patenterteilung: **02.08.2023**
(21) Anmeldenummer: 19000064.6
(22) Anmeldetag: 04.02.2019
(51) Int. Cl.: A41D 13/05, A61F 5/02

(54) **WIRBELSÄULENPROTEKTOR**
SPINE PROTECTOR
DISPOSITIF DE PROTECTION DE LA COLONNE VERTÉBRALE

(30) Priorität: 05.03.2018 AT 600372018; 17.10.2018 AT 601792018
(43) Veröffentlichungstag der Anmeldung: 11.09.2019
(73) Patentinhaber: O'NEAL Europe GmbH & Co. KG, 71665 Vaihingen an der Enz (DE)
(72) Erfinder: Saier, Thomas Adam, 9020 Klagenfurt (AT); Rafolt, Dietmar, 8043 Graz (AT); Falk, Eduard, 8010 Graz (AT); Meixner, Luis, 8010 Graz (AT)
(74) Vertreter: Meissner Bolte Partnerschaft mbB

(56) Entgegenhaltungen:
- WO-A1-2011/047647
- WO-A1-2011/150401
- DE-U1-202006 004 993
- GB-A- 2 159 058
- US-A1- 2005 197 607
- US-A1- 2018 027 893

## Beschreibung

Den Gegenstand dieser Erfindung bildet ein tragbarer Wirbelsäulenprotektor zur Verhinderung übermäßiger physiologischer Auslenkungen der Wirbelsäule eines Nutzers (einer Person). Der Wirbelsäulenprotektor weist dabei eine untere Anbindungsstruktur auf, die im Hüftbereich des Nutzers angeordnet wird und den Wirbelsäulenprotektor im Hüftbereich mit dem Nutzer verbindet. Ebenso weist der Wirbelsäulenprotektor eine obere Anbindungsstruktur auf, die im Schulter- oder Brustbereich des Nutzers angeordnet wird und die den Wirbelsäulenprotektor mit dem Oberkörper des Nutzers verbindet.

Im Sport und in der Arbeitswelt kommt es sehr häufig zu Verletzungen der Wirbelsäule, wenn das normale physiologische Bewegungsmaß überschritten wird. Der Großteil der Querschnittverletzungen wird dabei durch eine übermäßige Rotationsbewegung der Wirbelsäule hervorgerufen. Aus diesem Grund wurden bereits entsprechende am Körper getragene Protektoren entwickelt, die nicht nur wie herkömmliche Rückenprotektoren vor Schlagtraumen schützen, sondern auch übermäßige Kräfte bei extremen Auslenkungen aufnehmen. So beschreibt beispielsweise die WO 2016015070 A1 einen Wirbelsäulenprotektor, bei dem Bewegungen der Wirbelsäule in einem bestimmten, biomechanisch normalen Bereich ungehindert zugelassen werden, bei dem jedoch bei versuchter Überschreitung dieses Bereiches übermäßige Bewegungen idealerweise vollständig blockiert werden. Dies geschieht im Wesentlichen durch mechanische Anschläge und die Straffung von flexiblen Verbindungsbändern und -seilen. Auch wenn dieser Protektor sehr wirkungsvoll ist, so besitzt er doch einen recht komplizierten Aufbau und ist dementsprechend teuer in der Herstellung.

Die WO 2011/047647 A1 offenbart eine Vorrichtung zur Erfassung und/oder Beeinflussung der Körperhaltung bestehend aus zwei bevorzugt elastischen Spanngurten, die im Schulter und Beckenbereich befestigt sind.

Die GB 2 159 058 A beschreibt eine Stützvorrichtung für die Wirbelsäule durch die die Schultern nach hinten gezogen werden und so eine aufrechte Haltung gewährleistet wird. Der Benutzer kann sich dabei leicht nach vorne und zur Seite bewegen, wohingegen Rotationsbewegungen weitgehend verhindert werden.

Der Erfindung liegt daher die Aufgabe zugrunde, einen Wirbelsäulenprotektor bereitzustellen, der übermäßige Rotationsbewegungen der Wirbelsäule blockiert. Er soll also eine übermäßige Rotation der Schulterregion gegenüber der Hüftregion verhindert. Außerdem soll dieser Wirbelsäulenprotektor unkritische Bewegungen der Wirbelsäule nicht oder kaum behindern. Zudem soll er einen möglichst einfachen Aufbau aufweisen, damit er kostengünstig hergestellt werden kann.

Gelöst wird diese Aufgabe durch einen Wirbelsäulenprotektor gemäß Patentanspruch 1. Gemäß dieser Variante ist dabei die untere Anbindungsstruktur des Wirbelsäulenprotektors mit der oberen Anbindungsstruktur über zumindest zwei Bänder verbunden. Die beiden Bänder sind dabei vor der neutralen, seitlichen Biegelinie des Nutzers mit der unteren Anbindungsstruktur verbunden. Das erste Band verläuft entlang der Taille des Nutzers auf der rechten Seite A schräg nach oben zum Rücken des Nutzers und das zweite Band verläuft entlang der Taille des Nutzers auf der linken Seite B schräg nach oben zum Rücken des Nutzers. Das erste Band ist dabei mit der oberen Anbindungsstruktur im Bereich der linken Schulter oder des linken Brustbereiches des Nutzers verbunden. Das zweite Band ist mit der oberen Anbindungsstruktur im Bereich der rechten Schulter oder des rechten Brustbereiches des Nutzers verbunden, sodass sich die beiden Bänder im Rückenbereich des Nutzers kreuzen. Erfindungsgemäß ist die obere Anbindungsstruktur in Form eines Brustgurtes ausgeführt oder sie weist zwei Armschlaufen auf, die miteinander verbunden sind.

Unter dem Begriff "Bänder" werden im Sinne dieser Erfindung auch Seile, Gurte, Schläuche oder ähnliche Verbindungselemente verstanden.

Bei einer Rotation der Wirbelsäule, also bei einer Rotation der Hüftregion gegenüber der Schulterregion, wird erfindungsgemäß eines der beiden Bänder gestrafft bzw. gespannt und dadurch eine weitere Verdrehung der Wirbelsäule verhindert. Dadurch kann die Wirbelsäule vor einer übermäßigen physiologischen Auslenkung bzw. Rotation, die zu Verletzungen führen würde, bewahrt werden. Über die Länge der Bänder könnte beispielsweise der mögliche Drehwinkel der Wirbelsäule eingestellt werden. Mit einer definierten Bandlänge ist somit eine definierte Auslenkung der Wirbelsäule gegeben, bei der dann eine Rotationsblockade einsetzt.

Vorzugsweise ist die Bandlänge einstellbar, sodass sich dadurch auch eine Einstellbarkeit des Blockadewinkels ergibt. So eignet sich der Wirbelsäulenprotektor auch zur Anwendung im Rehabereich.

Wenn die beiden Bänder an der Schulterbefestigung und an der Hüftbefestigung an der Rückseite hinter der neutralen Biegelinie, die sich seitlich am Körper des Nutzers befindet, angebracht sind, dann würden beide Bänder beim Vorbeugen des Körpers gestrafft werden. Der mögliche Rotationsbereich würde sich dadurch entsprechend abhängig von der Vorbeugung des Nutzers einengen. Wenn der Körper nach hinten gebogen wird (Reklination bzw. Dorsalextension) würden die beiden Bänder gelockert werden und somit die Rotationsblockade unwirksam werden. Damit dies nicht geschieht, sind gemäß der Erfindung die beiden Bänder mit der unteren Anbindungsstruktur vor der seitlichen neutralen Biegelinie des Nutzers befestigt. Unter der seitlichen neutralen Biegelinie des Nutzers wird jene Linie verstanden, die bei einer Biegung des Oberkörpers nach vorne (Flexion) oder einer Biegung nach hinten (Extension) weder eine Dehnung noch eine Verkürzung erfährt, d.h. zwar gebogen wird, aber in der Länge konstant bleibt. Diese Linie verläuft beiderseits von der Achsel zum Hüftknochen. Jede Verbindungslinie, die nach rückwärts verschoben ist, erfährt bei Flexion eine Dehnung, bei Extension ein Verkürzung, ungeachtet ob die Verbindungslinie zwischen Schulter und Hüfte vertikal oder schräg (gekreuzt) verläuft.

Entsprechend umgekehrt verhält es sich bei einer Verschiebung der Verbindungslinie nach vorne. Erfindungsgemäß wird beispielsweise beim Vorbiegen des Oberkörpers die Straffung der Bänder auf der Hinterseite mit einer Lockerung der Bänder auf der Vorderseite des Nutzers kompensiert, wobei aber gleichzeitig die Blockadefunktion hinsichtlich der Rotationsbewegung der Wirbelsäule erhalten bleibt.

Bei einer Beugung des Nutzers nach vorne wird also eine Geometrieverlängerung auf der Körperrückseite mit einer Geometrieverkürzung auf der Körpervorderseite kompensiert. Die Bänder behindern daher eine Flexion beziehungsweise eine Dorsalextension nicht, sondern blockieren primär nur übermäßige Rotationsbewegungen der Wirbelsäule.

Da gemäß der Erfindung zumindest eine Befestigungsstelle pro Band vor der neutralen Biegelinie liegt, hat dies auch den Vorteil, dass die Steigung der Bänder (Diagonallinie bzw. Steigungslinie) geringer (flacher) ist und somit der bei übermäßigen Bewegungen auftretende Kraftvektor in Körperlängsachse geringer wird, wodurch die obere Anbindungsstruktur und die untere Anbindungsstruktur bei einer Rotationsbewegung weniger stark zueinander gezogen werden. Die Kraft in dem diagonal geführten Band setzt sich nämlich zusammen aus einer Kraft in horizontaler Richtung entlang des Umfanges, die für die Rotationsblockade verantwortlich ist und eine Kraft in vertikale Richtung entlang der Körperachse, die die Anbindungsstrukturen von Hüfte und Schulter zueinander zieht.

Es ist günstig, wenn die Bänder mit der unteren Anbindungsstruktur auf der Vorderseite des Nutzers im Hüft- bzw. Nabelbereich verbunden sind.

Erfindungsgemäß ist die untere Anbindungsstruktur als Hüftgurt bzw. als Beckengurt ausgeführt. Der Hüft- bzw. Beckengurt kann auch harte schalenförmige Elemente aufweisen, sodass eine formschlüssige Anbindung der unteren Anbindungsstruktur am Nutzer gewährleistet ist.

In einer bevorzugten Ausführungsform werden die beiden Bänder im vorderen Beckenbereich des Nutzers oder im hinteren Beckenbereich des Nutzers über ein Umlenksystem umgelenkt und bilden so den Hüft- bzw. Beckengurt. Bei einer Rotation der Wirbelsäule und einer damit verbundenen Straffung eines der beiden Bänder kommt es durch das Umlenksystem auch zu einer Straffung (zu einem Zusammenziehen) des Hüftgurtes. Die in dem gestrafften Band auftretenden Kräfte werden dabei zur Straffung des Hüftgurtes verwendet.

Das Umlenksystem für die beiden Bänder kann auch für jedes der beiden Bänder einen Blockiermechanismus aufweisen, der zwar eine Straffung des Hüftgurtes erlaubt, bei einer Lockerung des gestrafften Bandes jedoch blockiert und somit dafür sorgt, dass der Hüftgurt gestrafft bleibt. Bei dem Blockiermechanismus kann es sich beispielsweise um eine selbstsperrende Schnalle handeln. Selbstsperrende Schnallen erlauben das Durchziehen eines Bandes in eine Richtung und blockieren, wenn man versucht das Band in die andere Richtung hindurchzuziehen. Derartige selbstsperrende Schnallen sind bekannt und werden beispielsweise im Schließmechanismus von Flugzeuggurten oder als Gurtstraffmechanismus für Rucksäcke verwendet.

Es ist günstig, wenn die beiden Bänder aus einem dehnfesten Material bestehen.

Der obere Befestigungspunkt der beiden Bänder mit der oberen Anbindungsstruktur liegt vorzugsweise im Bereich des Sternums des Nutzers, sodass auch eine Lateralflexion (Seitwärtsneigung der Wirbelsäule) die Grenzen der Rotationsblockade nicht beeinflusst.

Es ist denkbar, dass die beiden Bänder in schlauchartigen Kanälen geführt werden. Diese können zusätzliche Gleitschichten, beispielsweise aus Teflon, zur Reduktion der Reibung zwischen dem jeweiligen Band und der Kanalwand beinhalten. Dabei können auch flüssige oder pulverförmige Gleitmittel verwendet werden.

Die beiden Bänder können auch elastische Elemente enthalten, sodass die Bänder mit einer gewissen leichten Vorspannung getragen werden können. Dadurch besitzen sie auch in der entspannten Situation eine definierte Position. Die elastischen Elemente sind dabei aber so ausgeführt, dass sie ab einer gewissen Dehnungsgrenze oder Dehnungsgeschwindigkeit kein elastisches Verhalten mehr aufweisen, sodass durch eine Straffung der Bänder eine übermäßige Rotationsbewegung der Wirbelsäule verhindert wird.

Die elastischen Elemente können beispielsweise mit einer nichtnewtonschen Flüssigkeit gefüllt sein, wodurch eine geschwindigkeitsabhängige Rotationsdämpfung realisiert werden kann. Die Viskosität nichtnewtonscher Fluide ändert sich insbesondere mit der Schergeschwindigkeit und/oder der Belastungsdauer. Verwendbar wären hierfür Fluide, deren Viskosität mit steigender Schergeschwindigkeit ansteigt. Diese Eigenschaft wird auch als Dilatanz bezeichnet. Ein bekanntes Beispiel für derartige Flüssigkeiten sind Stärkesuspensionen. Eine schnelle Krafteinwirkung führt dazu, dass sich die Mischung eher als Feststoff denn als Flüssigkeit verhält. Langsamere und sanftere Bewegungen belassen den Stoff im flüssigen Zustand.

Alternativ können die elastischen Elemente mit einer newtonschen Flüssigkeit gefüllt sein und eine interne Verwirbelungsstruktur aufweisen, sodass sich dadurch ein ähnliches Verhalten wie bei der Verwendung einer nichtnewtonschen Flüssigkeit ergibt.

Der Tragekomfort erhöht sich, wenn die obere Anbindungsstruktur zwei Armschlaufen aufweist, die miteinander verbunden sind. Die beiden Armschlaufen sind dabei fest oder lösbar miteinander verbunden.

Im Folgenden wird die Erfindung anhand von Zeichnungen beschrieben.
Die Fig. 1 bis 3 zeigen einen schematischen Aufbau des Wirbelsäulenprotektors;
Fig. 4 zeigt ein Ausführungsbeispiel des Wirbelsäulenprotektors mit Umlenksystem von hinten;
Fig. 5 zeigt ein weiteres Ausführungsbeispiel eines Wirbelsäulenprotektors mit Umlenksystem;
Fig. 6 zeigt eine sehr ausgereifte Ausführungsform des Wirbelsäulenprotektors;
Fig. 7 und Fig. 8 zeigen ein Detail eines Wirbelsäulenprotektors mit Gurtstraffmechanismus;
Figur 9 zeigt einen möglichen Schichtaufbau eines Wirbelsäulenprotektors;
Fig. 10 zeigt einen Wirbelsäulenprotektor mit in die Bänder integrierten elastischen Elementen;
Fig. 11 zeigt einen Wirbelsäulenprotektor, der in ein Shirt integriert ist und der nicht unter den Schutzbereich dieser Erfindung fällt;
Fig. 12 zeigt einen Wirbelsäulenprotektor, bei dem die obere Anbindungsstruktur aufgeklebt ist und der nicht unter den Schutzbereich dieser Erfindung fällt;
Fig. 13 zeigt einen Wirbelsäulenprotektor, bei dem sich die beiden Bänder an der Rumpfvorderseite kreuzen und der nicht unter den Schutzbereich dieser Erfindung fällt.

Gleiche Bezugszeichen in den einzelnen Figuren bezeichnen jeweils gleiche Bauteile. Die Bezeichnung "rechts" bezieht hier auf die rechte Körperseite A des Nutzers und die Bezeichnung "links" bezieht sich auf die linke Seite B des Nutzers.

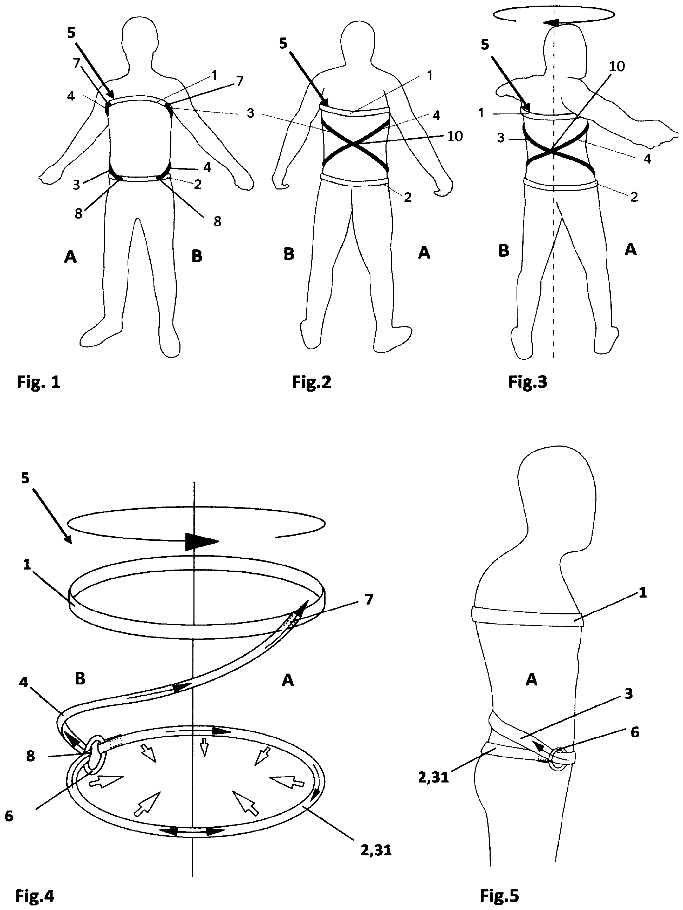

Fig.1 zeigt einen Nutzer, der ein Ausführungsbeispiel des Wirbelsäulenprotektors 5 trägt, von vorne. Der Wirbelsäulenprotektor 5 weist eine untere Anbindungsstruktur 2, in Form eines Hüftgurtes, und eine obere Anbindungsstruktur 1, hier in Form eines Brustgurtes, auf. Die untere Anbindungsstruktur 2 ist mit der oberen Anbindungsstruktur 1 über zwei Bänder 3, 4 verbunden. Das erste Band 3 ist dabei mit der unteren Anbindungsstruktur 2 über den unteren Befestigungspunkt 8 verbunden, der hier an der Vorderseite des Nutzers im Leistenbereich zwischen Beckenknochen und Bauchmitte liegt. Vom unteren Befestigungspunkt 8 aus läuft das erste Band 3 auf der rechten Seite A des Nutzers entlang der Taille schräg nach oben zum Rücken und ist mit der oberen Anbindungsstruktur 1 am oberen Befestigungspunkt 7, der hier im Bereich der linken Brust liegt, verbunden. Entsprechend verläuft das zweite Band 4 vom unteren Befestigungspunkt 8 entlang der Taille auf der linken Seite B des Nutzers schräg nach oben und ist mit der oberen Anbindungsstruktur 1 im oberen Befestigungspunkt 7 im Bereich der rechten Brust verbunden. Die beiden Bänder 3 und 4 müssen im Rahmen dieser Erfindung nicht einteilig sein, sie können auch Schnellverschlüsse zum Öffnen oder Schnallen zur Längeneinstellung beinhalten. In Figur 2 ist der Nutzer aus Fig. 1 von hinten dargestellt. Man erkennt dabei gut, dass sich die Bänder 3 und 4 im Rücken des Nutzers im Punkt 10 kreuzen. In diesem Kreuzungspunkt 10 sind die Bänder 3 und 4 nicht miteinander verbunden, sondern können sich relativ zueinander bewegen. Fig. 3 zeigt den Nutzer aus Figur 2 mit leicht nach rechts A verdrehtem Oberkörper. Bei dieser Bewegung wird das erste Band 3 gestrafft und das zweite Band 4 gelockert. Die Straffung des ersten Bandes 3 verhindert eine übermäßige Auslenkung bzw. Rotation der Wirbelsäule.

Fig. 4 zeigt ein Ausführungsbeispiel eines Wirbelsäulenprotektors 5 mit Umlenksystem 6 im Hüftbereich. Zur Erläuterung der Funktionsweise des Umlenksystems 6 ist hier zur besseren Übersichtlichkeit nur das zweite Band 4 dargestellt. Der Wirbelsäulenprotektor 5 ist hierbei von hinten dargestellt, wobei A die rechte Seite und B die linke Seite des Wirbelsäulenprotektors 5 (bezogen auf einen Nutzer) bezeichnet. Man erkennt hierbei, dass das zweite Band 4 im Anbindungspunkt 8 mit der unteren Anbindungsstruktur 2 durch das Umlenksystem 6, hier in Form eines Ringes, umgelenkt wird, um die Hüfte des Nutzers herumläuft und an seinem Ende mit dem Umlenksystem 6 fest verbunden ist. Das zweite Band 4 bildet somit auch den Hüftgurt 31. In Figur 4 ist eine Situation dargestellt, die auftritt, wenn ein Nutzer den Oberkörper nach links B dreht. Das zweite Band 4 wird bei dieser Bewegung gestrafft und dabei durch das Umlenksystem 6 nach oben gezogen. Dadurch zieht sich der Hüftgurt 31 zusammen, dies ist durch die Pfeile dargestellt.

Das Zusammenziehen des Hüftgurtes 31 bei einer Rotationsbewegung der Wirbelsäule hat mehrere Vorteile. Aus Komfortgründen sollte der Hüftgurt 31 bzw. die unter Anbindungsstruktur 2 nicht permanent straff sitzen. Durch das Umlenksystem 6 wird erst bei einer Rotation der Wirbelsäule der Hüftgurt 31 straffgezogen und dadurch ein guter Sitz verursacht. Eine gute Anbindung des Hüftgurtes 31 an den Nutzer ist sehr wichtig, da für eine zuverlässige Rotationsblockade ein Rutschen des Hüftgurtes 31 verhindert werden muss. Außerdem setzt der Körper des Nutzers dem Zusammenziehen des Hüftgurtes 31 eine Kraft entgegen, wodurch das Band 4 nicht beliebig weit durch das Umlenksystem 6 nach oben gezogen werden kann. Es wird mit immer größer werdenden Widerstand abgebremst. Dadurch wird auch die Rotationsbewegung der Wirbelsäule nicht ruckartig gestoppt, sondern gleichfalls mit einer immer größer werdenden Kraft abgebremst. Das Band 4 kann beispielsweise in einem Stück mit dem Hüftgurt 31 gefertigt werden. Fig. 4 stellt dabei die Funktionsweise für nur eine Rotationsrichtung dar. Wenn man diesen Mechanismus dann für beide Rotationsrichtungen verwendet, muss für das erste Band 3 ein zweiter Hüftgurt zum Einsatz kommen, mit einem analogen jedoch spiegelverkehrten Aufbau, wie das System für das zweite Band 4. Die untere Anbindungsstruktur 2 würde dann aus zwei Hüftgurten bestehen.

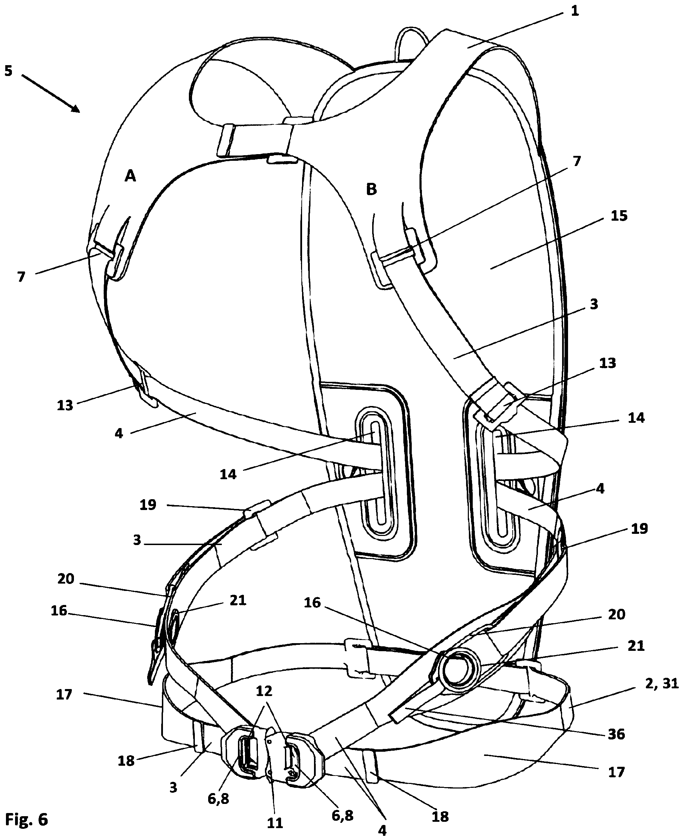

Sinnvollerweise wird aber nur ein Hüftgurt 31 verwendet, wobei im Hüftgurt 31 das Band 3 in das Band 4 übergeht, man erkennt dies beispielsweise in Figur 6. Dadurch wird sowohl bei einer Rotationsbewegung nach links B als auch bei einer Rotationsbewegung nach rechts A der Hüftgurt 31 gespannt. Bei dieser Lösung werden die beiden Umlenksysteme 6 jedoch vorzugsweise nicht durch einen einfachen Ring gebildet, sondern weisen auch jeweils einen Blockiermechanismus 12 auf. Dies ist sinnvoll, damit nicht beim Spannen des Hüftgurtes 31 über das erste Umlenksystem 6 das Band durch das zweite Umlenksystem 6 nachgezogen werden kann. Das würde ein Spannen des Hüftgurtes 31 verhindern und die Funktion als Rotationsblockade negativ beeinflussen.

Das Umlenksystem 6 kann auch als Rolle oder als Schnalle ausgeführt sein. Die Übertragung der Kraft in den Bändern 3, 4 auf den Hüftgurt 31 könnte auch mittels einer Hebelübersetzung ausgeführt werden, indem zum Beispiel der Hüftgurt 31 aufgetrennt ist und mit einem Hebelarm über Drehpunkte verbunden ist. Dieser wird dann von dem gespannten Band 3 oder 4 betätigt.

Das Hebelelement kann beispielsweise auch aus einer Platte bestehen, die mehrere Löcher für verschiedenen Positionen der Drehpunkte beinhaltet, wodurch die Hebelverhältnisse und auch der Diagonalwinkel der Bänder 3, 4 eingestellt werden können.

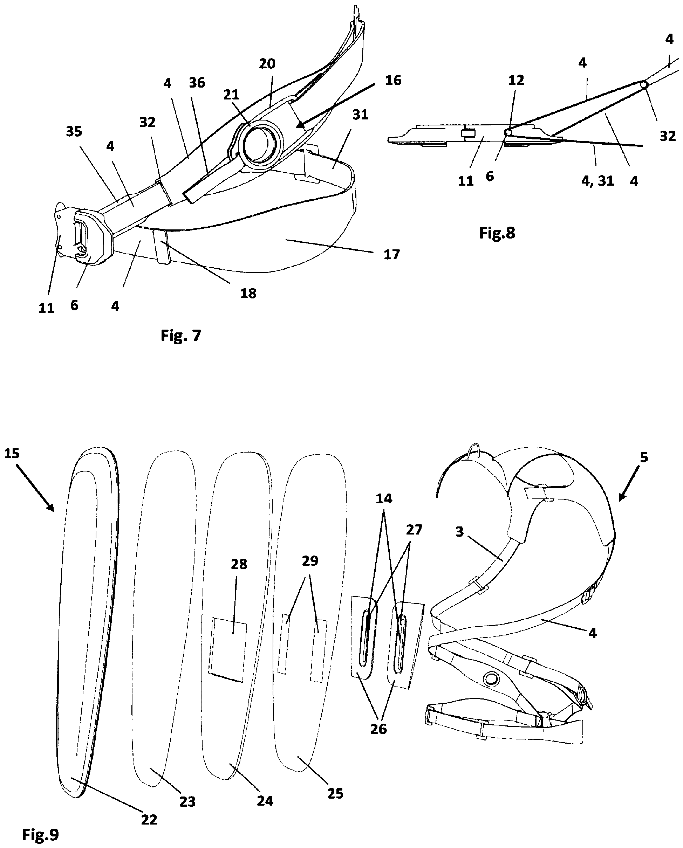

Zur Übersetzung der Zugkräfte können auch ein oder mehrere Flaschenzüge in die Bänder 3, 4 eingebaut werden, wie dies in Fig. 8 dargestellt ist.

Fig. 5 zeigt einen Wirbelsäulenprotektor 5 an einem Nutzer angebracht. Bei dieser möglichen Ausführungsform wird das erste Band 3 durch das Umlenksystem 6 im Gegensatz zur Fig. 4 nur leicht umgelenkt und läuft im Wesentlichen in der gleichen Richtung weiter. Es umschlingt den unteren Rumpfbereich und ist an seinem Ende mit dem Umlenksystem 6 verbunden. Auch hier kommt es durch die Straffung des ersten Bandes 3 zu einem Zusammenziehen des Hüftgurtes 31. Das zweite Band 4 ist hier nicht dargestellt.

Fig. 6 zeigt eine Gesamtansicht eines bereits sehr ausgereiften Wirbelsäulenprotektors 5. Das erste Band 3 ist mit der oberen Anbindungsstruktur (Brustbefestigung) 1 auf der linken Körperseite B am oberen Befestigungspunkt 7 in Form einer Schnalle verbunden und verläuft über die Spannvorrichtung 13 zur Körperhinterseite. Dieser Wirbelsäulenprotektor 5 hat auch einen zusätzlichen Rückenprotektor 15, der einen Aufprallschutz darstellt. Im Rückenprotektor 15 befinden sich Kanäle 14, in denen sich die Bänder 3 und 4 kreuzen. Vom Rückenprotektor 15 verläuft das erste Band 3 über den Schnellverschluss 16 weiter zum Umlenksystem 6 im Schnappverschluss 11. Dort wird das erste Band 3 umgelenkt und bildet den Hüft- bzw. Beckengurt 31. Der Beckengurt 31 erweitert sich im Bereich 17 des Beckenknochens und ist dort mit einer härteren Schicht zwecks besseren Formschlusses versehen. Der Hüftgurt 31 verjüngt sich nach der Beckenknochenkrümmung wieder und verläuft über den hinteren Hüftgurtteil 31 zur gegenüberliegenden linken Seite B des Beckens und ist dort im Bereich 17 des Beckenknochens ebenfalls verbreitert. Da das System symmetrisch aufgebaut ist, wiederholen sich die einzelnen Abschnitte in verkehrter Reihenfolge. Das erste Band 3 geht in das zweite Band 4 über, welches über das Umlenksystem 6 des Schnappverschlusses 11 zum linken Schnellverschluss 16 und von dort weiter zum Rückenprotektor 15 verläuft und sich dort mit dem ersten Band 3 kreuzt. Danach verläuft das zweite Band 4 über die Spannvorrichtung 13 zum oberen Befestigungspunkt 7 im Bereich der rechten Brust. Die obere Anbindungsstruktur 1 ist mit dem Rückenprotektor 15 im Nackenbereich verbunden. Die Längeneinstellung der Bänder 3,4 erfolgt vorzugsweise über die Spannvorrichtungen 13 und die Verbindungsschnallen 19.

Auch beim Wirbelsäulenprotektor 5 gemäß Fig. 6 wird der Hüftgurt 31 durch die Straffung eines der beiden Bänder 3, 4 zusammengezogen. Diese Selbstspannung des Hüftgurtes 31 erfolgt durch die Verwendung eines Schnappverschlusses 11 mit beidseitigen asymmetrischen Feststellmechanismus, welcher ein dauerhaftes Festzurren des Gurtes ermöglicht. Dies erfolgt indem die Bänder 3 und 4 zur Spannseite des jeweiligen Blockiermechanismus 12 führen und den Hüftgurt 31 spannen können. Da sich der Hüftgurt 31 auf der Blockadeseite vom Blockiermechanismus 12 befinden, bleibt die Hüftgurtspannung aufrecht, auch wenn sich die Bänder 3 und 4 wieder entspannen. Der Blockiermechanismus 12 besteht beispielsweise aus einer Rolle, über die jeweils die Bänder 3 und 4 umgelenkt werden (Umlenksystem 6). Diese Rolle kann dabei in eine Richtung ungehindert rotieren, sie blockiert jedoch bei einer Rotation in die andere Richtung. So können die Bänder 3,4 nach oben durch den jeweiligen Blockiermechanismus 12 gezogen werden, jedoch nicht nach unten. Als Blockiermechanismus 12 kann eine selbstsperrende Schnalle verwendet werden.

Da die beiden Blockiermechanismen 12 über den Weg entlang des Hüftumfanges quasi hintereinander geschalten sind, bewirkt eine Linksrotation wie auch eine Rechtsrotation im Falle einer übermäßigen Rotation ein Festzurren des Hüftgurtes 31. Bei einer Rechtsrotation des Oberkörpers (und/oder bei einer gleichzeitigen relativen Linksrotation der Hüfte) wird das Band 3 gestrafft und das Band 4 gelockert. Dabei wird über das rechte Umlenksystem 6 das Band 3 durch den rechten Blockiermechanismus 12 gezogen. Die Rolle des rechten Blockiermechansimus 12 rotiert dabei ohne zu blockieren. Der Hüftgurt zieht sich zusammen. Die entstehende erhöhte Kraft im Hüftgurt 31 wird auf den linken Teil des Schnappverschlusses 11 übertragen und trifft auf die Blockadefunktion des linken Blockiermechanismus 12, wodurch das Band 4 nicht nachgezogen werden kann. Dies ist die Bedingung für die symmetrische Funktionsweise des Wirbelsäulenprotektors 5. Der Körper des Nutzers setzt dem Zusammenziehen des Hüftgurtes 31 eine Kraft entgegen, wodurch das erste Band 3 letztendlich nicht mehr weiter durch das Umlenksystem 6 nach oben gezogen werden kann. Die Rotationsbewegung der Wirbelsäule wird gestoppt.

Einmal gestrafft, bleibt der Hüftgurt 31 in diesem Zustand, bis ein oder beide Blockiermechanismen 12 aktiv wieder gelöst werden. Dazu muss das Bandsystem 3, 4, 31 entlastet werden, wofür der Schnellverschluss 16 (einzeln oder beide) und in der Folge der Schnappverschluss 11 selbst gelöst wird. Dann können die Blockiermechanismen 12 gekippt werden, wodurch der Hüftgurt 31 wieder entlastet werden kann.

In der gegenwärtigen Ausführung wird der Schnellverschluss 16 dahingehend realisiert, dass ein Bügel 20 aus einem flexiblen Material, wie beispielsweise Gummi, über eine vorzugsweise runde oder ovale Verankerung 21 gezogen wird, wobei der Bügel 20 in einer Rille der Verankerung 21 sicher liegen kann. Zwecks besserer Bedienbarkeit kann der Bügel 20 mittels einer Lasche 36 über die Verankerung 21 gezogen werden. Der Vorteil des Schnellverschlusses 16 ist, dass für die jeweilige Person nur eine einmalige Einstellung des Wirbelsäulenprotektors 5 erfolgen muss, beispielsweise über die Spannvorrichtungen 13, und die Lockerung des Bandsystems über einen Schnellverschluss 16 erfolgt, der einfach aufgemacht wird, wenn der Träger den Wirbelsäulenprotektor 5 ablegen will oder aus Bequemlichkeitsgründen lockern möchte. Der Schnellverschluss 16 kann auf beiden Seiten oder auch nur auf einer Seite realisiert werden, da es zur Lockerung des Schnappverschlusses 11 genügt, auf nur einer Seite das Band 3,4 zu erweitern.

Es können auch andere Schließmechanismen anstatt dem Ringverschluss 20, 21 zum Einsatz kommen, wie beispielsweise Umlegeschnappverschlüsse, wie sie von Uhrenarmbändern bekannt sind.

Mit diesem Umlenkmechanismus ist es möglich, mit nur mäßig angespanntem Hüftgurt 31 Sport auszuüben, wodurch der Wirbelsäulenprotektor 5 nicht den Komfort einschränkt. Je extremer jemand einen Sport ausübt, was mitunter mit größeren Verdrehungswinkeln einhergeht, umso mehr wird der Hüftgurt 31 gespannt.

Im Hüftbereich ist die Anbindung eines Wirbelsäulenprotektors 5 im Vergleich zur Schulterregion weit schwieriger, sofern auf Oberschenkelgurte beispielsweise in Form von Sitzgurten verzichtet werden soll. Ein Rutschen des Hüftgurtes 31 um die Körperachse sollte unbedingt vermieden werden, damit der Wirbelsäulenprotektor 5 seine Wirksamkeit behält. In der gegenständlichen Erfindung wird eine formschlüssige Anbindung an den Hüftbereich vorgestellt, die eine verbesserte Kraftübertragung bei Rotationskräften bietet. Ausgenützt wird die Kontur des Beckenknochens, die einen Kraftschluss zulässt. Wesentlich ist, dass um den Hüftknochenbereich ein möglichst starres Material in Form von Schalen verwendet wird. Um diese Schalen herum kann ein Hüftgurt 31 aus verschiedenen vorzugsweise dehnungsfesten Material gelegt werden, sodass die Schalen gut an die Hüftkontur angekoppelt wird. Die zweigeteilten Strukturen lassen sich besser an verschiedenen Konfektionsgrößen anpassen. Zwischen den Hüftschalen und der Hüfte kann eine Polsterung mit nichtnewtonscher Flüssigkeit oder einem anderen viskosen Material angebracht sein, die dem Tragekomfort dient, da diese weicher ist und sich besser an die Hüftstruktur anschmiegt, bei schnellen Bewegungen aber entsprechend den Prinzip nichtnewtonscher Flüssigkeiten hart ist und einen direkteren Formschluss erzeugt. Der gegenständliche Wirbelsäulenprotektor 5 kann aber auch mit einem Sitzgurt oder mit Oberschenkelgurten in beliebiger Form, die zur Stabilisierung des Hüftgurtes 31 beitragen, kombiniert werden.

Aufgrund der erwünschten Reibung und des Formschlusses des Hüftgurtes 31 an der Hüfte gelangt nur ein Teil der auf der einen Seite eingeleiteten Kraft zur Komplementärseite des Schnappverschlusses 11. Somit kann der unidirektionale Blockiermechanismus 12 des Schnappverschlusses 11 auch durch eine (symmetrische) Reibungsumlenkung ersetzt werden. Diese muss so dimensioniert werden, dass die ankommende Kraftkomponente kleiner ist als die Reibungskraft, um ein Nachziehen, des lockeren Bandes (Band 4 im Falle der Rechtsrotation) zu verhindern. Dieser Reibungswert muss auch zur Straffung des Hüftgurtes 31 überwunden werden, was kein Nachteil ist, da im Falle eines Unfalles sowieso weit höhere Kraftwerte im Band 3,4 auftreten. Somit könnte ein kostengünstigerer Umlenkmechanismus ausgewählt werden. Der Reibungswert in der Bandumlenkung kann symmetrisch oder asymmetrisch sein.

Der Verschluss am Hüftgurt 31 kann wie dargestellt als Schnappverschluss 11 ausgeführt sein, er kann aber durch einen Klett - oder Magnetverschluss realisiert werden.

Mit speziellen Herstellungsverfahren können auch Bänder 3, 4 hergestellt werden, die in der Breite variieren können, ohne dass sich die Anzahl der Längsfäden ändert. Das hat einen besonders positiven Effekt auf die Festigkeit. In der gegenständlichen Erfindung können solche Bänder 3, 4 an mehreren Stellen eingesetzt werden. Zum einen können die Bänder 3,4 im Bereich des Schnellverschlusses 16 verbreitert sein, da sonst eine Schwächung infolge des Loches für den Einhängering 21 erfolgen würde.

Ein ähnlicher Bandaufbau kann für den Hüftgurt 31 verwendet werden. Im breiteren Bereich 17 des Hüftgurtes 31 können formsteifere Teile eingesetzt werden, wie beispielsweise Kunststoff- oder Aluminiumplatten oder Schalen, die einen besseren Formschluss am Beckenknochen ermöglichen. Diese steiferen Teile können am Hüftgurt 31 angeklebt, angenietet oder mit beliebig andern Techniken verbunden werden.

Figur 7 zeigt nochmals den unteren Teil des Wirbelsäulenprotektors 5. Die beiden optionalen Stopperelemente 18 verhindern, dass der Hüftgurt 31 zu sehr asymmetrisch verrutschen bzw. verstellt werden kann. Diese Stopperelemente 18 können auch verschiebbar und verriegelbar ausgeführt werden. Ebenso besser sichtbar ist der Schnellverschluss 16. Die Ausführung mit dem Schnellverschluss 16 ist zwar aufwendiger, bieten aber den Vorteil, dass hiermit nur zwei Positionen möglich sind, nämlich die gelockerte und die gespannte Position. Die individuellen Einstellungen bleiben dadurch, wenn sie einmal eingestellt sind, erhalten.

In Figur 7 ist im Abschnitt zwischen dem Umlenksystem 6 und dem Schnellverschluss 16 eine Umlenkrolle 32 angeordnet. Das Band 4 wird hier durch eine Führung 35 zum Umlenksystem 6 geführt. Das Band 4 ist am Schnappverschluss 11 befestigt, läuft hoch zur Umlenkrolle 32 und wird von da wieder nach unten zum Umlenksystem 6, also zur Rolle des Blockiermechanismus 12, geführt. Von der Umlenkrolle 32 nach oben zur oberen Anbindungsstruktur 1 läuft ebenfalls ein Band 4. Dadurch entsteht ein einfacher Flaschenzug. Das Grundprinzip dieses Flaschenzuges ist nochmals in Figur 8 dargestellt. Das erste Band 3 bzw. das zweite Band 4 kann also auch durch mehrere hintereinandergeschaltete Einzelbänder realisiert werden.

Die Flaschenzugfunktion kann in beide Richtungen eingesetzt werden. Entweder es erfolgt eine Kraftübersetzung, sodass der Hüftgurt 31 mit der doppelten Kraft gespannt wird, oder eine Wegübersetzung, die den Hüftgurt 31 doppelt so viel verkürzt, wie das jeweilige Band 3, 4 zieht. Damit ist die Wirkungsgrenze des Wirbelsäulenprotektors 5 schärfer gemacht. Nachteil eines Flaschenzugmechanismus ist, dass komplementär die Kraft oder der Weg halbiert wird.

Figur 9 zeigt den Schichtaufbau eines Wirbelsäulenprotektors 5 mit einem Rückenprotektor 15. Der Rückenprotektor 15 besteht aus einem äußeren geschäumten oder festen Teil 22, einer vorzugsweise dichten Folie 23, einer Abstandsmatte 24 mit der Ausnehmung 28 für die Bänder 3, 4, einer vorzugsweisen textile Schicht 25 mit den Ausnehmungen 29 durch welche die Bänder 3, 4 gefädelt werden und einer Verstärkungen 26, beispielsweise aus Kunststoff oder Leder zur Führung der Bänder 3, 4 mit den Ausnehmungen 27.

Vor dem Schäumen oder Zusammennähen werden die Schichten 23, 24, 25 zusammengenäht oder geklebt und die Führungsteile 26 auf der Schicht 25 befestigt. Alternativ können die Führungsteile 26 auch zwischen die Schichten 24 und 25 eingefügt werden.

Die Bänder 3, 4 treten somit durch die Einlässe 14 in den Rückenprotektor 15 ein und kreuzen sich am Rücken in der Ausnehmung 28.

Die Schulteranbindung der oberen Anbindungsstruktur 1 ist im Gegensatz zur Hüftbefestigung durch die anatomisch bedingte bessere Befestigungsmöglichkeit bezüglich Verdrehung und einer Verschiebung längs der Körperachse relativ stabil.

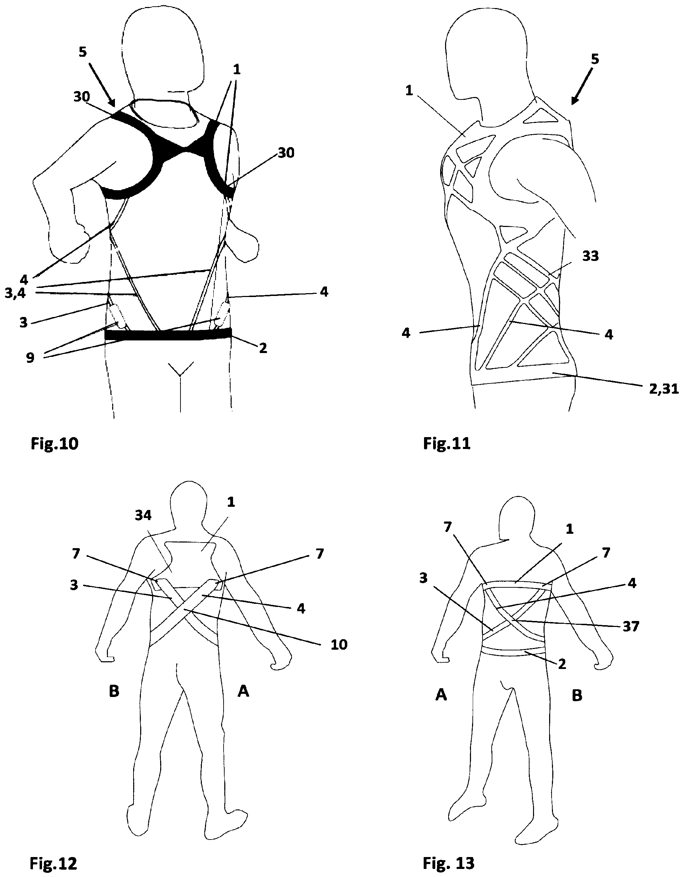

Eine Schulterbefestigung kann beispielsweise aus zwei Armschlaufen 30 bestehen, in die man mit den Armen hineinschlüpft und die vorn und hinten direkt zusammenstoßen, oder die, wie dies Fig. 10 dargestellt ist, über Querteile aus flexiblen oder steifen Material miteinander verbunden sind. Die Verbinder können Trennstellen aus beispielsweise Schnallen, Schnappverschlüsse oder Klettverbindungen (Velcro) beinhalten.

Die Befestigung des Wirbelsäulenprotektors 5 am Nutzer kann beispielsweise auch so ausgeführt sein, dass die Bänder 3,4 dafür direkt verwendet werden, indem diese um Schulter und Oberarm in einer Schleife herumgeführt werden. Die obere Anbindungsstruktur 1 wird dann zumindest teilweise durch die Bänder 3,4 gebildet.

Die Bänder 3, 4 des Wirbelsäulenprotektors 5 können auch in die Bänder integrierte elastischen Elemente 9 aufweisen. Dies ist beispielhaft in Figur 10 angedeutet. Bei diesen elastischen Elementen 9 kann es sich um schlauchförmige Hohlräume handeln, die von einem dehnbaren Material gebildet werden und die mit einer nichtnewtonschen Flüssigkeit gefüllt sind. Dadurch kann eine geschwindigkeitsabhängige Kraftübertragung in den Bändern 3, 4 erreicht werden. Bei langsamen Drehbewegungen der Wirbelsäule wird eines der beiden Bänder 3 oder 4 langsam gespannt und die mit der nichtnewtonschen Flüssigkeit gefüllten elastischen Elemente 9 können sich verformen, also in die Länge gezogen werden. Das Bandsystem verhält sich da elastisch. Wenn es jedoch zu einer schlagartigen Straffung eines der Bänder 3, 4 kommt, verhält sich die nichtnewtonsche Flüssigkeit in den elastischen Elementen 9 wie ein Festkörper. Die elastischen Elemente 9 werden dadurch kaum verformt, die Bänder 3, 4 verhalten sich unelastisch und die Rotationsblockade ist wirksam.

Die elastischen Elemente 9 der Bänder 3, 4 können auch ein dehnfestes textiles Blockadeband aufweisen, das auf ein elastisches, leicht gespanntes Trägerband segmentweise befestigt, beispielsweise genäht oder geklebt wird, und zwar so, dass bei Entlastung des Trägerbandes, das Blockadeband Falten wirft, wodurch das Gesamtbandsystem eine leichte Vorspannung erfährt und gleichzeitig ab einer gewissen Dehnung dehnfest ist. Die Hohlräume der Falten können auch mit einer nichtnewtonschen Flüssigkeit gefüllt sein. Bei einer Dehnung des jeweiligen Bandes 3, 4 sind die Falten einer starken Geometrieverformung unterworfen und widersetzen sich schnellen Änderungen und behindern somit übermäßig schnelle Rotationen.

Fig. 11 zeigt nicht erfindungsgemäße Anwendungsbeispiele in dem der Wirbelsäulenprotektor 5 in ein Kleidungsstück, beispielsweise in ein Kompressionsshirt integriert wurde. Hierbei sind pro Seite mehrere Bänder 3,4 vorgesehen, wobei die Bänder 3 bzw. 4 pro Seite über Verbindungselemente 33 miteinander verbunden sind.

Fig. 12 zeigt ein nicht erfindungsgemäßes Ausführungsbeispiel für einen Wirbelsäulenprotektor 5. Hier wird die obere Anbindungsstruktur 1 durch eine aufgeklebte Anbindung 34 realisiert.

Fig. 13 zeigt eine nicht erfindungsgemäße Variante. Gemäß dieser Variante sind die beiden Bänder 3, 4 zumindest mit einer der beiden Anbindungsstrukturen 1, 2 hinter der neutralen, seitlichen Biegelinie des Nutzers verbunden und kreuzen sich im vorderen Rumpfbereich. In der dargestellten Ausführungsform ist das erste Band 3 hinter der seitlichen, neutralen Biegelinie im rechten hinteren Beckenbereich mit der unteren Anbindungsstruktur 2 verbunden. Das erste Band 3 verläuft dann auf der rechten Seite A um die Taille des Nutzers herum schräg nach oben zur Vorderseite und ist mit der oberen Anbindungsstruktur 1 im Bereich der linken Schulter verbunden. In analoger Weise ist das zweite Band 4 im linken hinteren Beckenbereich mit der unteren Anbindungsstruktur 2 verbunden, verläuft auf der linken Seite B um die Taille des Nutzers schräg nach oben zur oberen Anbindungsstruktur 1 und ist mit dieser im rechten Schulterbereich über den oberen Befestigungspunkt 7 verbunden. Die beiden Bänder 3, 4 kreuzen sich hier im vorderen Rumpfbereich (Bauchbereich) des Nutzers. Da hier die beiden Bänder 3, 4 mit der unteren Anbindungsstruktur hinter der seitlichen, neutralen Biegelinie des Nutzers verbunden sind, wird auch hier beim Vorbeugen des Oberkörpers die Straffung der Bänder 3, 4 auf der Körperrückseite mit einer Lockerung der Bänder 3, 4 auf der Vorderseite des Nutzers kompensiert, sodass die Blockadefunktion hinsichtlich der Rotationsbewegung der Wirbelsäule erhalten bleibt. Das Bandsystem gemäß dieser Variante kann natürlich auch die Elemente der oben beschriebenen ersten Variante aufweisen. Im Speziellen kann es ein Umlenksystem 6, vorzugsweise mit Blockiermechanismus 12, einen Schnellverschluss 16 oder die beschriebenen elastischen Elemente 9 aufweisen.

Der in den Fig. 1 bis 10 gezeigte Wirbelsäulenprotektor 5, könnte auch so beschrieben werden, dass er aus einer unteren kreisförmigen Anbindungsstruktur 2 und einer oberen Anbindungsstruktur 1 besteht, die auch kreisförmig ist oder Armschlaufen aufweist. Die untere Anbindungsstruktur 2 ist mit der oberen Anbindungsstruktur 1 über zumindest 2 Bänder 3 und 4 verbunden. Die obere Anbindungsstruktur 1 ist von der unteren Anbindungsstruktur 2 in vertikaler Richtung beabstandet. Die beiden Bänder 3, 4 sind mit der unteren Anbindungsstruktur 2 im vorderen Bereich verbunden, verlaufen schräg nach oben, kreuzen sich und sind dann mit der oberen Anbindungsstruktur 1 verbunden.

Der Wirbelsäulenprotektor 5 lässt sich auch mit herkömmlichen Rückenprotektoren verschiedener Hersteller kombinieren. Der Wirbelsäulenprotektor 5 gemäß Fig. 6 kann auch mit einem modifizierten Schulterteil alleine (d.h. ohne Schlagschutzrückenprotektor) zur Rotationsbegrenzung eingesetzt werden. Letztere Anwendung ist auch im medizinischen Bereich beispielsweise in der Rehabilitation nach Wirbelsäulenverletzungen möglich.

### Bezugszeichen

1 Obere Anbindungsstruktur
2 Untere Anbindungsstruktur
3 Erstes Band
4 Zweites Band
5 Wirbelsäulenprotektor
6 Umlenksystem
7 oberer Befestigungspunkt
8 unterer Befestigungspunkt
9 elastische Elemente
10 Kreuzungspunkt der Bänder 3,4 am Rücken
11Schnappverschluss
12 Blockiermechanismus
13 Spannvorrichtung
14 Einlässe/ Kanäle
15 Rückenprotektor
16 Schnellverschluss
17 Erweiterung des Becken- bzw. Hüftgurtes
18 Stopperelemente
19 Verbindungsschnalle
20 Bügel
21Verankerung, Ringverschluss, Einhängering
22 Äußerer Teil des Rückenprotektors
23 Folie
24 Abstandsmatte
25 Textile Schicht
26 Verstärkungen
27 Ausnehmungen
28 Ausnehmungen
29 Ausnehmungen
30 Armschlaufen
31 Hüftgurt
32 Umlenkrollen
33Verbindungselemente
34 Aufgeklebte Anbindung 1
35 Führung
36 Lasche
37 Kreuzungspunkt der Bänder 3, 4 an der Rumpfvorderseite
   A rechte Seite des Nutzers (vom Nutzer aus betrachtet)
   B linke Seite des Nutzers (vom Nutzer aus betrachtet)
33 Verbindungselemente
34 Aufgeklebte Anbindung 1
35 Führung
36 Lasche
37 Kreuzungspunkt der Bänder 3, 4 an der Rumpfvorderseite
   A rechte Seite des Nutzers (vom Nutzer aus betrachtet)
   B linke Seite des Nutzers (vom Nutzer aus betrachtet)

## Patentansprüche

1. Tragbarer Wirbelsäulenprotektor (5) zur Verhinderung übermäßiger physiologischer Auslenkungen einer Wirbelsäule eines Nutzers, wobei der Wirbelsäulenprotektor (5) eine untere Anbindungsstruktur (2) aufweist, die als Hüftgurt bzw. Beckengurt (31) ausgeführt ist, der im Hüftbereich des Nutzers angeordnet werden kann und wobei der Wirbelsäulenprotektor (5) eine obere Anbindungsstruktur (1) aufweist, die im Schulter- oder Brustbereich des Nutzers angeordnet werden kann, wobei die untere Anbindungsstruktur (2) mit der oberen Anbindungsstruktur (1) über zumindest zwei Bänder (3, 4) verbunden ist, wobei die zumindest zwei Bänder (3, 4) mit der unteren Anbindungsstruktur (2) vor der neutralen, seitlichen Biegelinie des Nutzers verbunden sind, wobei das erste Band (3) entlang der Taille des Nutzers auf der rechten Seite (A) schräg nach oben zum Rücken des Nutzers verläuft und das zweite Band (4) entlang der Taille des Nutzers auf der linken Seite (B) schräg nach oben zum Rücken des Nutzers verläuft, wobei das erste Band (3) mit der oberen Anbindungsstruktur (1) im Bereich der linken Schulter oder des linken Brustbereiches des Nutzers verbunden ist und wobei das zweite Band (4) mit der oberen Anbindungsstruktur (1) im Bereich der rechten Schulter oder des rechten Brustbereiches des Nutzers verbunden ist, sodass sich die beiden Bänder (3, 4) im Rückenbereich des Nutzers kreuzen, **dadurch gekennzeichnet, dass** die obere Anbindungsstruktur (1) in Form eines Brustgurtes ausgeführt ist oder, dass die obere Anbindungsstruktur (1) zwei Armschlaufen (30) aufweist, die miteinander verbunden sind.

2. Wirbelsäulenprotektor (5) nach Anspruch 1, **dadurch gekennzeichnet, dass** die beiden Bänder (3, 4) mit der unteren Anbindungsstruktur (2) im vorderen Beckenbereich des Nutzers verbunden sind.

3. Wirbelsäulenprotektor (5) nach Anspruch 1 oder 2, **dadurch gekennzeichnet, dass** die beiden Bänder (3, 4) im vorderen Beckenbereich des Nutzers oder im hinteren Beckenbereich des Nutzers über ein Umlenksystem (6) umgelenkt werden und so den Hüftgurt (31) bilden, sodass es bei einer Rotation der Wirbelsäule und einer damit verbundenen Straffung eines Bandes (3, 4) auch zu einer Straffung des Hüftgurtes (31) kommt.

4. Wirbelsäulenprotektor (5) nach Anspruch 3, **dadurch gekennzeichnet, dass** das Umlenksystem (6) für die beiden Bänder (3, 4) jeweils einen Blockiermechanismus (12) aufweist, der eine Straffung des Hüftgurtes (31) erlaubt, bei einer Lockerung des gestrafften Bandes (3, 4) jedoch blockiert und somit der Hüftgurt (31) gestrafft bleibt.

5. Wirbelsäulenprotektor (5) nach Anspruch 4, **dadurch gekennzeichnet, dass** der Blockiermechanismus (12) durch eine selbstsperrende Schnalle gebildet wird.

6. Wirbelsäulenprotektor (5) nach einem der Ansprüche 1 bis 5, **dadurch gekennzeichnet, dass** die beiden Bänder (3, 4) einen Schnellverschluss (16) aufweisen, durch den sie geöffnet werden können.

7. Wirbelsäulenprotektor (5) nach einem der Ansprüche 1 bis 6, **dadurch gekennzeichnet, dass** der Hüftgurt (31) harte schalenförmige Elemente aufweist, durch die er formschlüssig am Nutzer anbringbar ist.

8. Wirbelsäulenprotektor (5) nach einem der Ansprüche 1 bis 7, **dadurch gekennzeichnet, dass** der Hüftgurt (31) eine Polsterung enthält, die mit einer nichtnewtonschen Flüssigkeit gefüllt ist.

9. Wirbelsäulenprotektor (5) nach einem der Ansprüche 1 bis 8, **dadurch gekennzeichnet, dass** die beiden Bänder (3, 4) aus einem dehnfesten Material bestehen.

10. Wirbelsäulenprotektor nach Anspruch 1, **dadurch gekennzeichnet, dass** der Befestigungspunkt (7) der beiden Bänder (3, 4) mit der oberen Anbindungsstruktur (1) im Bereich des Sternums des Nutzers liegt.

11. Wirbelsäulenprotektor (5) nach einem der Ansprüche 1 bis 8, **dadurch gekennzeichnet, dass** die beiden Bänder (3, 4) elastische Elemente (9) enthalten.

12. Wirbelsäulenprotektor (5) nach Anspruch 11, **dadurch gekennzeichnet, dass** die elastischen Elemente (9) mit einer nichtnewtonschen Flüssigkeit gefüllt sind.

## Claims

1. Portable spine protector (5) to prevent excessive physiological deflections of a user's spine, where the spine protector (5) comprises a lower attachment structure (2) designed as a hip belt or pelvic belt (31) that can be placed in the user's hip region and where the spine protector (5) comprises an upper attachment structure (1) that can be placed in the user's shoulder or chest region, the lower attachment structure (2) being connected to the upper attachment structure (1) by at least two straps (3, 4), and the at least two straps (3, 4) being connected to the lower attachment structure (2) in front of the user's neutral lateral bending line, the first strap (3) running along the user's waist on the right-hand side (A) diagonally upwards to the user's back and the second strap (4) running along the user's waist on the left-hand side (B) diagonally upwards to the user's back, wherein the first strap (3) is connected to the upper attachment structure (1) in the region of the user's left shoulder or left chest, and wherein the second strap (4) is connected to the upper attachment structure (1) in the region of the user's right shoulder or right chest, so that the two straps (3, 4) cross over in the user's back region, **characterised in that** the upper attachment structure (1) is designed as chest strap or **in that** the upper attachment structure (1) has two slings (30) that are connected to one another.

2. Spine Protector (5) according to Claim 1, **characterised in that** the two straps (3, 4) are connected to the lower attachment structure (2) in the user's anterior pelvic region.

3. Spine Protector (5) according to Claim 1 or 2, **characterised in that** the two straps (3, 4) in the user's anterior pelvic region or his/her posterior pelvic region are deflected via a deflection system (6) and thus form the hip belt (31), so that a rotation of the spine and a tightening of one strap (3, 4) associated therewith also causes the hip belt (31) to tighten.

4. Spine Protector (5) according to Claim 3, **characterised in that** the deflection system (6) for the two straps (3, 4) in each case comprises a locking mechanism (12) which allows the hip belt (31) to be tightened but which locks when the tightened strap (3, 4) is loosened so that the hip belt (31) remains tightened.

5. Spine protector (5) according to Claim 4, **characterised in that** the locking mechanism (12) is formed by a self-locking buckle.

6. Spine protector (5) according to any one of Claims 1 to 5, **characterised in that** the two straps (3, 4) have a quick-release fastener (16) by means of which they can be opened.

7. Spine protector (5) according to any one of claims 1 to 6, **characterised in that** the hip belt (31) comprises shell-shaped, hard elements by means of which it can be attached to the user in a form-fitting manner.

8. Spine protector (5) according to any one of Claims 1 to 7, **characterised in that** the hip belt (31) includes padding filled with a non-Newtonian fluid.

9. Spine protector (5) according to any one of Claims 1 to 8, **characterised in that** the two straps (3, 4) are made of a stretch-resistant material.

10. Spine protector (5) according to Claim 1, **characterised in that** the attachment point (7) of the two straps (3, 4) to the upper attachment structure (1) is located in the region of the user's sternum.

11. Spine protector (5) according to any one of Claims 1 to 8, **characterised in that** the two straps (3, 4) contain elastic elements (9).

12. Spine protector (5) according to Claim 11, **characterised in that** the elastic elements (9) are filled with a non-Newtonian fluid.

## Revendications

1. Dispositif de protection de la colonne vertébrale portable (5) pour prévenir les déflexions physiologiques excessives de la colonne vertébrale d'un utilisateur, le dispositif de protection de la colonne vertébrale (5) comprenant une structure de fixation inférieure (2) conçue comme une ceinture de hanche ou une ceinture pelvienne (31) qui peut être placée dans la région de la hanche de l'utilisateur, et le dispositif de protection de la colonne vertébrale (5) comprenant une structure de fixation supérieure (1) qui peut être placée au niveau de l'épaule ou de la poitrine de l'utilisateur, la structure de fixation inférieure (2) étant reliée à la structure de fixation supérieure (1) par au moins deux sangles (3, 4), et les au moins deux sangles (3, 4) étant reliées à la structure de fixation inférieure (2) en avant de la ligne de flexion latérale neutre de l'utilisateur, la première sangle (3) longeant la taille de l'utilisateur sur le côté droit (A) en diagonale vers le haut jusqu'au dos de l'utilisateur, et la deuxième sangle (4) longeant la taille de l'utilisateur sur le côté gauche (B) en diagonale vers le haut jusqu'au dos de l'utilisateur, la première sangle (3) étant reliée à la structure de fixation supérieure (1) au niveau de l'épaule gauche ou de la poitrine gauche de l'utilisateur, et la deuxième sangle (4) étant reliée à la structure de fixation supérieure (1) au niveau de l'épaule droite ou de la poitrine droite de l'utilisateur, de sorte que les deux sangles (3, 4) se croisent dans la région du dos de l'utilisateur, **caractérisé en ce que** la structure de fixation supérieure (1) est conçue comme une sangle de poitrine ou **en ce que** la structure de fixation supérieure (1) comporte deux courroies (30) reliées l'une à l'autre.

2. Dispositif de protection de la colonne vertébrale (5) selon la revendication 1, **caractérisé en ce que** les deux sangles (3, 4) sont reliées à la structure de fixation inférieure (2) dans la région pelvienne antérieure de l'utilisateur.

3. Dispositif de protection de la colonne vertébrale (5) selon la revendication 1 ou 2, **caractérisé en ce que** les deux sangles (3, 4) dans la région pelvienne antérieure de l'utilisateur ou dans sa région pelvienne postérieure sont défléchies par un système de déflexion (6) et forment ainsi la ceinture de hanche (31), de sorte qu'une rotation de la colonne vertébrale et un serrage d'une sangle (3, 4) associés entraînent également un serrage de la ceinture de hanche (31).

4. Dispositif de protection de la colonne vertébrale (5) selon la revendication 3, **caractérisé en ce que** le système de déflexion (6) des deux sangles (3, 4) comprend dans chaque cas un mécanisme de blocage (12) qui permet de serrer la ceinture de hanche (31) mais qui se bloque lorsque la sangle serrée (3, 4) est relâchée, de sorte que la ceinture de hanche (31) reste serrée.

5. Dispositif de protection de la colonne vertébrale (5) selon la revendication 4, **caractérisé en ce que** le mécanisme de blocage (12) est constitué d'une boucle autobloquante.

6. Dispositif de protection de la colonne vertébrale (5) selon l'une quelconque des revendications 1 à 5, **caractérisé en ce que** les deux sangles (3, 4) sont munies d'une fermeture rapide (16) permettant de les ouvrir.

7. Dispositif de protection de la colonne vertébrale (5) selon l'une quelconque des revendications 1 à 6, **caractérisée en ce que** la ceinture de hanche (31) comprend des éléments durs en forme de coquille qui permettent de la fixer à l'utilisateur de manière à ce qu'elle épouse la forme de ce dernier.

8. Dispositif de protection de la colonne vertébrale (5) selon l'une quelconque des revendications 1 à 7, **caractérisé en ce que** la ceinture de hanche (31) comprend un rembourrage rempli d'un fluide non newtonien.

9. Dispositif de protection de la colonne vertébrale (5) selon l'une quelconque des revendications 1 à 8, **caractérisé en ce que** les deux sangles (3, 4) sont fabriquées dans un matériau résistant à l'étirement.

10. Dispositif de protection de la colonne vertébrale (5) selon la revendication 1, **caractérisé en ce que** le point de fixation (7) des deux sangles (3, 4) à la structure de fixation supérieure (1) est situé au niveau du sternum de l'utilisateur.

11. Dispositif de protection de la colonne vertébrale (5) selon l'une quelconque des revendications 1 à 8, **caractérisé en ce que** les deux sangles (3, 4) contiennent des éléments élastiques (9).

12. Dispositif de protection de la colonne vertébrale (5) selon la revendication 11, **caractérisé en ce que** les éléments élastiques (9) sont remplis d'un fluide non newtonien.
